# EUROPEAN PATENT APPLICATION

(11) **EP 2 712 657 A1**
(43) Date of publication of application: **02.04.2014**
(21) Application number: 13189937.9
(22) Date of filing: 01.12.2010
(51) Int. Cl.: A61Q 11/00, A61K 36/9068

(54) **Oral compositions containing extracts of zingiber officinale and related methods**

(30) Priority: 04.12.2009 US 266685 P
(62) Divisional of application: 10787233.5
(71) Applicant: Colgate-Palmolive Company, New York NY 10022-7499 (US)
(72) Inventor: Trivedi, Harsh M., Hillsborough, NJ New Jersey 08844 (US); Gittins, Elizabeth K., Stewartsville, NJ New Jersey 08886 (US)
(74) Representative: Jenkins, Peter David

(57) **Abstract**

Described herein are compositions comprising a combination of extracts, and methods of preparing and using the same.

## Description

### CROSS REFERENCE TO RELATED APPLICATIONS

This application claims priority to U.S. Provisional Patent Application No. 61/266,685, filed on 4 December 2010, which is incorporated herein by reference.

### BACKGROUND

Dentifrice compositions are widely used in order to provide oral health. Dentifrices in the form of toothpaste, mouth rinses, chewing gums, edible strips, powders, foams, and the like have been formulated with a wide variety of active materials that provide a number of benefits to the user. Among these benefits are antimicrobial, anti-inflammatory, and antioxidant properties. These properties of dentifrices make them useful therapeutic agents to prevent or treat a number of oral health conditions such as cavities, gingivitis, plaque, tartar, periodontal disease, and the like.

Gingivitis is the inflammation or infection of the gums and the alveolar bones that support the teeth. Gingivitis is generally believed to be caused by bacteria in the mouth (particularly the bacteria instigated in plaque formation) and the toxins formed as by-products from the bacteria. The toxins are believed to instigate oral tissue inflammation within the mouth. Periodontitis is a progressively worsened state of disease as compared to gingivitis, where the gums are inflamed and begin to recede from the teeth and pockets form, which ultimately may result in destruction of the bone and periodontal ligament. Bacterial infections of the structures that support the dentition can include gingivitis and periodontitis, but may also include infections of the bone, for example the mandibles as a result of surgical intervention. Further, oral tissue inflammation can be caused by surgery, localized injury, trauma, necrosis, improper oral hygiene or various systemic origins.

It is generally believed that the cellular components implicated by these diseases and conditions include epithelial tissue, gingival fibroblasts, and circulating leukocytes, all of which contribute to the host response to pathogenic factors generated by the bacteria. The most common bacterial pathogens implicated in these oral infections are *Streptococci* spp. *(e.g., S. mutans*), *Porphyromonas* spp., *Actinobacillus* spp., *Bacteroides* spp., *and Staphylococci* spp., *Fusobacterium nucleatum, Veillonella parvula, Actinomyces naeslundii, and Porphyromonas gingivalis.* Although the bacterial infection is often the etiological event in many of these oral diseases, the pathogenesis of the disease is mediated by the host response. Circulating polymorphonuclear neutrophils (PMNs) are largely responsible for the hyperactivity found at sites of infection. Typically PMNs and other cellular mediators of inflammation become hyperfunctional and release toxic chemicals that are partly responsible for the destruction of tissue surrounding the foci of infection.

Thus, bacterial infection of the oral tissue stimulates the host's immune response and diminishes the healing process by up-regulating inflammatory mediators that cause significant tissue damage. One class of mediators extensively studied for their effect on the inflammatory response is the arachidonic acid metabolites namely prostaglandins and leukotrienes, that are produced through the cyclooxygenase or lipoxygenase enzyme pathways. These metabolites have been implicated as the prime mediators in gingivitis, periodontitis, osteomyelitis and other inflammatory diseases.

There are a variety of compositions described in the art for preventing and treating oral inflammation as a result of bacterial infection. In particular, to prevent the accumulation of inflammatory mediators derived from arachidonic acid pathway, non-steroidal anti-inflammatory drugs (NSAIDs) have been used successfully to treat patients suffering from periodontal disease and inflammatory diseases that are caused by arachidonic acid metabolites. Experimental and clinical data have shown that indomethacin, flurbiprofen, ketoprofen, ibuprofen, naproxen, and meclofenamic acid have significant ameliorative effects against alveolar bone loss, and reduction of prostaglandins and leukotrienes in dental disease models. However, one major disadvantage to the regular use of NSAIDs is the potential development of heartburn, gastric ulcers, gastrointestinal bleeding, and toxicity.

Other treatment methods include the use of antimicrobial therapeutics and antibiotics to eliminate the underlying infection. These treatments operate to reduce the source of irritants (bacteria), but are slow to affect the host immune response to the toxins secreted by the bacteria. In addition, certain antibiotics and other antimicrobial therapeutics potentially cause ulceration of oral mucous membranes, induction of desquamative gingivitis, discoloration, the potential for antibiotic resistance after prolonged usage, as well as exacerbation of tissue inflammation due to irritation.

Essential oils have been used in dentifrice compositions, primarily as flavorants. Many essential oils are oils of plants, but the composition of an oil of a plant is differs a great deal from an extract of that plant.

*Zingiber officinale* (ginger) has been in medical use since ancient times, and has a wide range of properties alleged to be useful for a wide range of ailments. *Zingiber officinale* has been investigated for anti-inflammatory, analgesic, antipyretic, antimicrobial and hypoglycaemic activities. Mascolo, N., et al., "Ethnopharmacologic Investigation of ginger (Zingiber officinale), J. Ethnopharmacol., 27, pp. 129-140 (Nov. 1989). *Zingiber officinale* has been reported to present antibacterial efficacy on four respiratory tract pathogens (*Staphylococcu aureus, Streptococcus pyogenes, Streptococcus pneumoniae,* and *Haemophilus influenzae*). Akoachere, J.F., et al., "Antibacterial effect of Zingiber officinale and Garcinia kola on respiratory tract pathogens," East Afr. Med. J., 79, pp 588-592 (Nov. 2002). Certain components of ginger, specifically, gingerol, have been reported to possess antimicrobial activity against oral bacteria. Park, et al., "Antibacterial activity of (10)-Gingerol and (12)-Gingerol isolated from Ginger Rhizome against periodontal bacteria," J. Phytother. Res., 22(11), pp. 1446-1449, (Nov. 2008). Some extracts of ginger have been reported to have anti-fungal activity. Atai, et al., "Inhibitory Effect of Ginger Extract on Candida albicans," Am. J. App. Sciences, 6(6), pp. 1067-1069 (2009).

U.S. Patent Nos. 6,264,926, and 7,083,779 discloses that some tooth powders containing *Zingiber officinale* are not very effective, and can be harmful to gums and teeth, as well as toxic. U.S. Patent No. 4,423,030 discloses a high sensation oleoresin prepared by solvent extraction from dried rhizomes of ginger (*zingiber officinale*) as useful as an essential oil flavorant in a dental cream or mouthwash.

U.S. Patent Application Publication No. 2009/0131364 discloses bioactive extracts of *zingiber officinale* that are useful in treating oxidative stress induced diseases, such as ulcers. U.S. Patent Application Publication No. 2007/011652 discloses red tooth powders containing botanical extracts, which may include an extract of *zingiber officinale.* Other documents disclose the anti-fungal, anti-inflammatory, or other health benefit effects of various extracts of *zingiber officinale.* See, e.g., U.S. Patent Nos. 6,946,153 and 6,274,177, and U.S. Patent Application Publication No. 2009/0104293.

While some have reported health benefits from the use of extracts from *Zingiber officinale,* these uses do not suggest that extracts of *Zingiber officinale* would provide any oral care benefits in combination with other natural extracts, other than perhaps use as a flavor-enhacing agent. There is a need to provide natural supplements that provide antibacterial, anti-inflammatory, as well as antioxidant effects to the oral cavity.

### SUMMARY

It has now been found that addition of extract of *Zingiber officinale* to various dentifrice compositions results in tooth paste, mouth rinses, gums, mouth strips, beads, and other compositions that are suitable for treating and preventing a variety of oral disease including gingivitis, plaque build-up, and the like. The extract of *Zingiber officinale,* believed to contain varying amounts, *inter alia,* of one or more of bisaboline, cineol, phelladrene, citral, borneal, citronellal, geramial, linalool, limonene, zingiberol, zingiberine, camphene, gingerol, shogaol, dehydrogingerdione, zingerone, zingibain, vitamin B6, vitamin C, calcium, magnesium, phosphorus, potassium, linoleic acid, pectic polysaccharides (rhammose, arabinose, xylose, mannose, galactose, glucose and the like), gallic acid, tannic acid, gentisic acid, protocatechuic acid, Vanillic acid, caffeic acid, syringic acid, cinnamic acid, and mixtures thereof, and other beneficial chemicals, can be added to dentifrice compositions so that the amount delivered to the oral cavity upon use is effective to provide an antibacterial, antioxidant, and/or anti-inflammatory effect. In various embodiments, the components of extract of *Zingiber officinale,* are combined with natural extracts other than extracts of *Zingiber officinale* to provide enhanced activity.

It has been found that dentifrices formulated with the extract of *Zingiber officinale,* in combination with other natural extracts, exhibit antimicrobial, anti-inflammatory, and/or antioxidant properties, as well as being effective in treating xerostomia, without the need for an additional antibacterial agent.

In accordance with a feature of an embodiment, there is provided an oral composition comprising an extract from *Zingiber officinale,* an orally acceptable carrier, and a natural extract other than the extract from *Zingiber officinale.* In another feature of an embodiment, there is provided a method of treating soft tissue in the oral cavity comprising administering to soft tissue in the oral cavity a composition comprising an extract from *Zingiber officinale,* an orally acceptable carrier, and a natural extract other than the extract from *Zingiber officinale.*

Further areas of applicability of the present invention will become apparent from the detailed description provided hereinafter. It should be understood that the detailed description and specific examples, while indicating the preferred embodiment of the invention, are intended for purposes of illustration only and are not intended to limit the scope of the invention.

### DETAILED DESCRIPTION

As used throughout, ranges are used as shorthand for describing each and every value that is within the range. Any value within the range can be selected as the terminus of the range. In addition, all references cited herein are hereby incorporated by reference in their entireties. In the event of a conflict in a definition in the present disclosure and that of a cited reference, the present disclosure controls. In addition, the compositions and the methods may comprise, consist essentially of, or consist of the elements described therein.

Unless otherwise specified, all percentages and amounts expressed herein and elsewhere in the specification should be understood to refer to percentages by weight. The amounts given are based on the active weight of the material. The recitation of a specific value herein is intended to denote that value, plus or minus a degree of variability to account for errors in measurements. For example, an amount of 10% may include 9.5% or 10.5%, given the degree of error in measurement that will be appreciated and understood by those having ordinary skill in the art.

As used herein, "antibacterial activity" herein means activity as determined by any generally accepted *in vitro* or *in vivo* antibacterial assay or test. "Anti-inflammatory activity" herein means activity as determined by any generally accepted *in vitro* or *in vivo* assay or test, for example an assay or test for inhibition of prostaglandin production or cyclooxygenase activity. "Antioxidant activity" herein means activity as determined by any generally accepted in vitro or in vivo antioxidant assay or test.

An "oral surface" herein encompasses any soft or hard surface within the mouth including surfaces of the tongue, hard and soft palate, buccal mucosa, gums and dental surfaces. A "dental surface" herein is a surface of a natural tooth or a hard surface of artificial dentition including a crown, cap, filling, bridge, denture, dental implant and the like. The term "inhibiting" herein with respect to a condition such as inflammation in an oral tissue encompasses prevention, suppression, reduction in extent or severity, or amelioration of the condition.

The expression "natural extract" as used herein denotes any extract that is obtained from a natural source, such as a plant, fruit, tree, and the like. Non-limiting examples of natural extracts include extracts of oregano, magnolia, rosemary, *Camellia,* morin, *myristic fragrans, Zizyphus joazeiro, Punica granatum, Garcinia mangostana L,* Jabara, *Azadirachta indica, Acacia, Oolong tea, Juglans regia, Zanthoxylum alantum, Mimusops elengi, Hibiscus abelmoschus,Ayurvedic,Carapa procera, Khaya senegalensis, Salvadora persica,Cucurbitaceae (Citrullus colocynthis),* and the like. Many such extracts are disclosed in U.S. Patent Nos. 6,264,926 and 7,083,779, and U.S. Patent Application Publication Nos. 2009/0087501, and 2007/0116652.

An oral care composition of the present invention can take any form suitable for application to an oral surface. In various illustrative embodiments the composition can be a liquid solution suitable for irrigating, rinsing or spraying; a dentifrice such as a powder, toothpaste or dental gel; a periodontal gel; a liquid suitable for painting a dental surface (*e.g*., a liquid whitener); a chewing gum; a dissolvable, partially dissolvable or non-dissolvable film or strip or patch, (*e.g.*, a whitening strip); a bead (e.g., composition encapsulated in gelatin), a wafer; a wipe or towelette; an implant; a mouthrinse, a foam, dental floss; *etc.* The composition can contain active and/or carrier ingredients additional to those recited above.

In certain embodiments the composition is adapted for application to an oral surface of a small domestic animal, for example a cat or a dog. Such a composition is typically edible or chewable by the animal, and can take the form, for example, of a cat or dog food, treat or toy.

Classification herein of an ingredient as an active agent or a carrier ingredient is made for clarity and convenience, and no inference should be drawn that a particular ingredient necessarily functions in the composition in accordance with its classification herein. Furthermore, a particular ingredient can serve a plurality of functions, thus disclosure of an ingredient herein as exemplifying one functional class does not exclude the possibility that it can also exemplify another functional class.

In one embodiment, a tooth paste composition is provided that contains at least an extract from *Zingiber officinale,* an orally acceptable carrier, and a natural extract other than the extract from *Zingiber officinale. Zingiber officinale* belongs to the family *Zingeberaceae,* is cultivated in various parts of the world especially India, China, Mexico etc. It is a major spice crop cultivated in India and marketed as fresh and dried spice. It is perennial small grassy plant grown in all season through out the year. Indian ginger is famous for its flavor, texture and taste. More than spices, ginger is considered as tastemaker, drug, appetizer and flavorant. Ginger products are available in a variety of forms like oils, oleoresins, fresh ginger in brine, pickle, candies, syrup etc. Bleached and unbleached powder forms of ginger also are available in the market. India has a predominant position in ginger production and export. In the world market, Indian ginger is popularly known as Cochin ginger and Calicut ginger. The principal buyers are the Middle East, USA, UK and Netherlands. Ginger is commonly used for abdominal bloating, coughing, vomiting, diarrhea, rheumatism etc.

Ginger is cultivated mainly for its root part or rhizome. The proximate chemical composition of ginger has been investigated and has been shown to contain approximately 1-4% of volatile oils, which are the medically active constituents of ginger (2009/0131364). The volatile oils are believed to consist of bisaboline, cineol, phelladrene, citral, borneal, citronellal, geramial, linalool, limonene, zingiberol, zingiberine, camphene etc. The oleoresin present in ginger is mainly gingerol and shogaol, although extracts also include dehydrogingerdione. The phenols detected in solvent extracts of ginger are mainly gingerol and zingerone. Zingibain a proteolytic enzyme is also present in ginger, in addition to other components such as vitamin B6, vitamin C, calcium, magnesium, phosphorus, potassium and linoleic acid etc. The pungency and aroma of ginger has been identified to be mainly due to gingerol, which contains alcohol group of the oleoresin, volatile oil respectively. This makes Ginger a free radical scavenger and its antimutagenic and anti-inflammatory properties, have been documented.

The extract of *Zingiber officinale* can be obtained in any of a variety of known methods of extraction. The extract is believed to contain any one or more of the following compounds, which itself, or in combination with other compounds found in the extract, can be used in the embodiments. Extracts of *Zingiber officinale* may include bisaboline, cineol, phelladrene, citral, borneal, citronellal, geramial, linalool, limonene, zingiberol, zingiberine, camphene, gingerol, shogaol, zingerone, zingibain, vitamin B6, vitamin C, calcium, magnesium, phosphorus, potassium, linoleic acid, pectic polysaccharides (rhammose, arabinose, xylose, mannose, galactose, glucose and the like), gallic acid, tannic acid, gentisic acid, protocatechuic acid, Vanillic acid, caffeic acid, syringic acid, cinnamic acid, and mixtures thereof.

In another embodiment, the invention provides a method for inhibiting bacterial growth and/or inflammation in the oral cavity of a subject animal. The method preferably is a method of treating soft tissue in the oral cavity comprising administering to soft tissue in the oral cavity a composition comprising an extract from *Zingiber officinale,* an orally acceptable carrier, and a natural extract other than the extract from *Zingiber officinale.*

In another embodiment, the invention provides mouth rinses or mouth washes comprising water, flavorants, and at least one hydric component such as ethanol, glycerol, and sorbitol together with an extract from *Zingiber officinale,* and at least one natural extract other than the extract from *Zingiber officinale.* In another embodiment, the invention provides a chewing gum comprising a gum base and flavorants in addition to an extract from *Zingiber officinale,* and a natural extract other than the extract from *Zingiber officinale.* In yet a further embodiment, edible strips are provided that contain film forming polymers and optionally flavorants in addition to an extract from *Zingiber officinale,* and a natural extract other than the extract from *Zingiber officinale.*

In one aspect, the composition contains a natural extract other than the extract from *Zingiber officinale.* Any suitable extract can be used so long as it enhances the antibacterial, anti-inflammatory, and antioxidant effects of the extract from *Zingiber officinale.* Suitable extracts include, for example, extracts of oregano, magnolia, cranberry, rosemary, *Camellia,* morin, *Myristica fragrans,, Zizyphus joazeiro, Punica granatum, Garcinia mangostana L.,* Jabara, *Azadirachta indica, Acacia, Oolong tea, Juglans regia, Zanthoxylum alantum, Mimusops elengi, Hibiscus abelmoschus, Ayurvedic, Carapa procera, Khaya senegalensis, Salvadora persica,Cucurbitaceae (Citrullus colocynthis),* and the like.

Particularly preferred extracts include extracts of oregano, magnolia, cranberry, rosemary, *Camellia,* morin, *Myristica fragrans, Zizyphus joazeiro, Punica granatum, Garcinia mangostana L.,* Jabara, *Azadirachta indica, Acacia, Oolong tea, Juglans regia, Zanthoxylum alantum, Mimusops elengi, Hibiscus abelmoschus, Ayurvedic, Carapa procera, Khaya senegalensis, Salvadora persica,Cucurbitaceae (Citrullus colocynthis), Acacia catechu, Acacia nilotica, Achyrathes aspera, Azadirachta indica, Aristolochia bracteolate, Cinnamomum camphora, Cinnamomum verum, Curcuma longa, Eucalyptus globulus, Ficus bengalensis, Juglans regia, Madhuca longifolia, Mimusops elengi, Ocimum sanctum,* Oolonga tea, Piper betel leaves, *Piper longum, Piper nigrum, Potentilla fulgens, Syzygium aromaticum, Spilanthes calva, Vaccinium macrocarpon, Zanthoxylum armatum,* and mixtures thereof.

Additional extracts can be selected from one or more plants of the following genera: *Origanum Thymus, Lavandula, Salvia, Melissa, Cuminum, Petroselinum, Calendula, Tagetes, Boswellia, Sambucus, Copaifera, Curcuma, Allium, Symphytum, Punica, Euterpe, Sophora, Rheum, Fagopyrum, Camellia, Coptis, Hydrastis, Mahonia, Phellodendron, Berberis, Xanthorhiza, Lonicera, Vaccinium, Cinnamomum, Vitis, Terminalia, Pinus, Albizia, Melia, Salvadora, Paullinia, Piper, Syzygium, Commiphora, Juglans, Scutellaria,* and Magnolia.

More specifically, the additional natural extract used in the compositions described herein can be extracted from plants of the following species: *Origanum vulgare, Origanum onites, Origanum majorana, Origanum heracleoticum, Thymus vulgaris L, Thymus citriodorus, Thymus pulegioides, Thymus x herba-barona, Thymus serpyllum, Lavandula angustifolia*/*officinalis, Lavandula stoechas, Lavandula dentate, Lavandula x intermedia, Lavandula multifida, Salvia officinalis, Salvia divinorum, Salvia apiana, Melissa officinalis, Cuminum cyminum, Petroselinum crispum, Calendula arvensis, Calendula maderensis, Calendula officinalis, Tagetes erecta, Tagetes minuta, Tagetes patula, Boswellia sacra, Boswellia frereana, Boswellia serrata, Boswellia papyrifera, Sambucus nigra, Sambucus melanocarpa, Sambucus racemosa, Copaifera langsdorfii, Curcuma longa, Allium sativu, Symphytum officinale, Punica granatum, Euterpe oleracea, Sophora flavescens, Rheum rhabarbarum, Rheum rhaponticum, Fagopyrum esculentum, Camellia sinensis, Coptis teeta, Hydrastis canadensis, Mahonia aquifolium, Phellodendron amurense, Berberis vulgaris, Xanthorhiza simplicissima, Lonicera ceprifoliu, Vaccinium macrocarpon, Cinnamomum zeylanicum Nees, Cinnamomum verum, Vitis Vinifera, Terminalia Bellerica, Pinus Pinaster, Albizia Lebbek, Melia Azadirachta, Salvadora persica, Paullinia cupana, Piper betle, Syzygium aromaticum, Commiphora myrrha, Juglans regia, Scutellaria baicalensis,* and *Magnolia officinalis.*

The additional natural extracts useful together with the *Zingiber officinale* extract also may be selected from one or more of the following natural extracts (common name included first, not italicized, followed by formal name(s) in italics): achyranthes, *Achyranthes aspera,* aloe, *Aloe spp.,* including *A. barbadensis, A. ferox* and *A. vera,* anise, *Pimpinella anisum,* aristolochia, *Aristolochia bracteolate,* arnica, *Arnica spp.,* including *A. fulgens,* banyan, *Ficus bengalensis,* bakula, *Mimusops elengi,* basil, *Ocimum basilicum* and *O. minimum,* betel, *Piper betle,* black pepper, *Piper nigrum,* camphor, *Cinnamomum camphora,* catechu, *Acacia catechu,* celandine, *Chelidonium spp.,* chamomile, *Matricaria chamomilla,* chebula, *Terminalia chebula,* Chinese skullcap, *Scutellaria baicalensis,* cinnamon, *Cinnamomum lourerii* and *C. zeylandicum,* citrus, *Citrus spp.,* including *C*. *aurantifolia, C. aurantium, C. limonum* and *C. sinensis,* clove, *Syzygium aromaticum,* dill, *Anethum spp.,* including *A. graveolens* and *A. sowa,* echinacea (coneflower), *Echinacea pallida,* eucalyptus, *Eucalyptus globulus,* fennel, *Foeniculum vulgare,* gardenia, *Gardenia jasminoides,* grape, *Vitis vinifera,* hop, *Humulus lupulus,* houttuynia, *Houttuynia cordata,* Indian mulberry, *Morinda citrifolia,* juniper, *Juniperus communis,* lemongrass, *Cymbopogon spp.,* including *C. citratus* and *C.flexuosus,* licorice, *Glycyrrhiza spp.,* including *G*. *glabra* and *G*. *uralensis,* long pepper (pipli), *Piper longum,* madhuca, *Madhuca longifolia,* magnolia, *Magnolia officinalis,* marigold, *Calendula officinalis,* mastic, *Pistacia lentiscus,* melilot, *Melilotus officinalis,* milfoil, *Achillea millefolium,* myrrh, *Commiphora spp.,* including *C. abyssinica* and *C. molmol,* neem (margosa), *Azadirachta indica,* neroli (bitter orange blossom), *Citrus aurantium,* nutmeg, *Myristica fragrans,* oak gall, *Quercus infectoria,* parsley, *Petroselinum sativum,* peelu, *Salvadora persica,* peppermint *Mentha piperita,* pine, *Pinus spp.,* including *P. palustris* and *P. sylvestris,* pomegranate, *Punica granatum,* prickly acacia (babul), *Acacia nilotica,* rhatany, *Krameria spp.,* including *K argentea* and *K triandra,* rosemary, *Rosmarinus officinalis,* saffron, *Crocus sativus,* sage, *Salvia spp.,* including *S. lavendulaefolia, S. officinalis* and *S*. *triloba,* sandalwood, *Santalum spp.,* including *S*. *album* and *S. spicatum,* spearmint *Mentha spicata,* spilanthes (akarkara), *Spilanthes calvi,* star anise, *Illicium verum,* tea *(*including green tea and oolong tea*)*, *Camellia sinensis,* thyme, *Thymus spp.,* including *T. serpyllum* and *T vulgaris,* tomar (prickly ash), *Zanthoxylum armatum,* tulsi (holy basil), *Ocimum sanctum,* turmeric, *Curcuma longa,* usnea, *Usnea barbata,* vajradanti, *Potentillafulgens,* walnut, *Juglans regia,* wintergreen, *Gaultheria procumbens,* and mixtures thereof.

As discussed herein, the additional natural extracts may be derived from or based upon compounds or extracts isolated from plants. The following plants each provide one or more active ingredients that are useful in an oral composition for one or more oral care benefits. For example, extract from *Romains officinalis* (rosemary) has an antibacterial and anti-inflammatory effect. Rosemary extract contains various organic and inorganic materials, including flavonoids, triterpenic and phenolic acids. Non-limiting examples of the useful organic compounds include 1,8-cineole, camphor, α-pinene, camosic acid, rosmarinic acid, ursolic acid, carnosol, and oleanolic acid. The discussion of active compounds contained herein in relation to various extracts includes those compounds that are believed to be efficacious in oral compositions; however, the lists of such compounds are non-exclusive and in some cases are yet to be identified or fully characterized, however, empirical observation demonstrates the desired effects. Furthermore, in various aspects, the entire extract including all compounds contained therein provides the most effective botanical active ingredient. Rosemary extracts for use in oral compositions are discussed in U.S. Patent Publication 2006/0134025 to Trivedi et al. and assigned to Colgate-Palmolive. The extracts of the leaves of rosemary plants are sold as rosemary extract by, for example, Sabinsa Corporation of Piscataway, N.J. Such compounds found in various plant-based extracts may be isolated from the extracts and used independently as botanical active ingredients. For example, camosic acid may be independently isolated and used in an oral composition, as it has been found to be efficacious against oral bacteria that cause cavities, gingivitis, and bad breath.

Other extracts useful in accordance with the present teachings include any suitable part of a plant from the *Lamiaceae* family, including those plants classified in the following genera: *Origanum, Thymus, Lavandula, Salvia, Perovskia, Phlomis,* or *Melissa.* For example, suitable extracts include those from *Origanum vulgare L.* (commonly known as "oregano", "wild oregano", or "wild marjoram"), including its sub-species (*Origanum vulgare ssp.*), *Origanum onites* (commonly known as "Italian oregano" or "pot marjoram"). *Origanum majorana* (commonly known as "marjoram" or "sweet marjoram") and *Origanum heracleoticum. Origanum vulgare* subspecies include *O. vulgare ssp. vulgare, O. vulgare ssp. viride,* and *O. vulgare ssp. hirtum* (commonly known as "Greek oregano" or "Wild oregano"). As used herein, the term "Oregano" encompasses all suitable species and sub-species of the genus *Origanum.* Oregano is believed to contain over 30 active compounds, including carvarcrol, thymol, and rosmarinic acid.

The genus *Thymus* (Thyme), also of the family *Lamiaceae,* includes over three hundred species and sub-species. Suitable extracts include those isolated from the following plants: *Thymus vulgaris L, T. citriodorus, T. pulegioides, T. x herba-barona,* and *T. serpyllum.* As used herein, the term "Thyme" encompasses all suitable species and sub-species of the genus *Thymus,* and extracts derived therefrom, which are believed to contain carvarcrol and thymol active compounds.

Other suitable extracts include those from the *Lavandula* (lavender) genus, which encompasses over 30 species. Suitable lavender species include *Lavandula angustifolia* (formerly known as *L. Officinalis L.*), *L. stoechas; L. dentate: L. x intermedia*; and *L. multifida.* Lavender extracts contain the active compounds linalyl acetate and linalool, among others. The term "Sage" as used herein generally includes plants of three genera of the *Lamiaceae* family, namely *Salvia, Perovski,* and *Phlomis.* In certain aspects, useful plants include *Salvia officinalis* (common sage), *S. divinorum* (diviner's sage); and *S*. *apiana* (white sage). Extracts from *S*. *officinalis* have antibiotic, antifungal, and astringent effects, among others. Another suitable extract is derived from the lemon balm plant (*Melissa Officinalis*), which has antibacterial and antiviral properties.

Further extracts useful in accordance with the present embodiments also include those derived from plants of the *Apiaceae family,* including *Cuminum* and *Petroselinum. Cuminum cyminum* (Cumin) contains various active compounds, including cuminaldehyde and pyrazines. *Petroselinum crispum* (parsley) includes apiol, furanocourmarin, and psoralen compounds. Cumin and parsley extracts have beneficial antioxidant activity, as well as other beneficial effects.

*Genera Calendula* and *Tagetes,* both commonly known as "marigold," are both of the family *Asteraceae.* The Calendula genus include many species and sub-species, including *Calendula arvensis* (field marigold); *C. maderensis* (Madeiran marigold); and *C. officinalis* (pot marigold). Calendula extracts contain various active compounds, including calendic acid. The *Tagetes* genus includes over sixty species and sub-species, including *Tagetes erecta; T. minuta, T. patula* and the like. Extracts of both *Calendula* and *Tagetes* have antioxidant and anti-inflammatory activity and are efficacious against oral bacteria that cause cavities, gingivitis and bad breath.

*Boswellia* is a genus of trees that produce extracts having anti-inflammatory properties, including boswellic acid compounds. For example, *Boswellia sacra, B. frereana; B. serrata;* and *B. papyrifera* and their sub-species produce suitable extracts. A useful active compound isolated from the Boswellia plant is acetyl keto .beta.-boswellic acid (AKBBA), for example, 3-acetyl 11-keto .beta.-boswellic acid, which exhibits antibacterial, anti-inflammatory and antioxidant activities. A commercially available B. serrata extract including a mixture of .beta.-boswellic and organic acids is available from Sabinsa Corp., as BOSWELLIN® CG.

*Sambucus* includes over thirty species and subspecies, which are commonly referred to as elderberry or elder. Various *Sambucus* species are suitable, including *Sambucus nigra* (common elder); *S. melanocarpa* (blackberry elder); *S*. *racemosa* (red-berried elder), among others. The elderberry extracts have been discovered to have antioxidant activity, and further, provide one or more of the following benefits in an oral composition: antibacterial, antioxidant, collagenase inhibition, sirtuins activation, and anti-inflammatory properties.

Extracts of *Copaifera langsdorfii* (copaiba balsam) are useful, as are *Curcuma longa* (tumeric), which includes the compounds curcumin, demethoxycurcumin, bis-demethoxycurcumin, and tetrahydrocurcuminoid. Additional suitable extracts include those isolated from *Allium sativum* (garlic) or other plants of the *Allium* genera. Garlic extracts contain allicin, alliin, ajoene, and other flavonoids, which provide antioxidant and/or anti-microbial benefits. Extracts from *Symphytum officinale* (comfrey) or other plants of the genus *Symphytum* are useful as anti-oxidants, anti-inflammatory, and/or antimicrobial agents; as are *Punica granatum* (pomegranate) extracts which include various antioxidant polyphenols, such as hydrolyzable tannins punicalagins; *Euterpe oleracea* (Acai palm), which contains resveratrol, anthocyanins, and various other flavonoid and flavonoid-like compounds, such as homoorientin, orientin, tasifolin, deoxyhexose, isovitexin, scoparin; *Sophora flavescens* extracts, which contain kurarinone as a bioactive flavonoid, which has anti-inflammatory and antibacterial function. Each of the extracts described above exhibits one or more antioxidant, anti-inflammatory, antiviral, and/or antibacterial properties. A representative structure of kurarinone is:

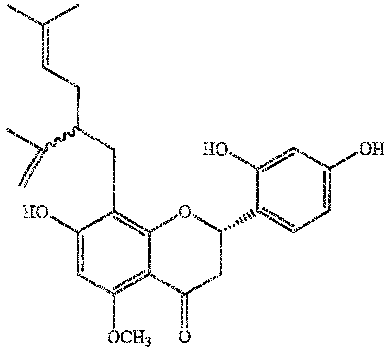

In certain aspects of the disclosure, the oral compositions optionally comprise a commercially available extract derived from *C. longa* that includes tetrahydrocurcuminoid, under the trade name SABIWHITE® available from Sabinsa Corp., which is believed to have the following representative structure:

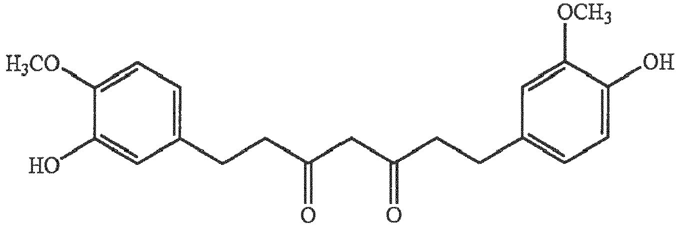

Various plant extracts contain the active compound rutin (quercetin-3-rutinoside) which is an antioxidant flavonoid glycoside (comprising the flavonol quercetin and the disaccharide rutinose) found in various plants of the Polygonaceae family, including the *Rheum* genus, including *Rheum rhabarbarum* and *R. rhaponticum* (garden rhubarb) and of the *Fagopyrum esculentum* Moench (buckwheat) plant. What is believed to be a representative structure is shown below:

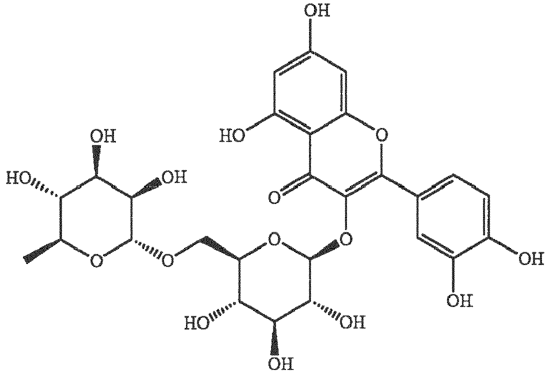

Rutin is believed to scavenge superoxide radicals, chelate metal ions, modulate bursts of neturophils, inhibit lipid peroxidation, maintain the biological antioxidant reduced glutathione, and has involvement in fenton reactions (which generate reactive oxygen species). Thus, rutin has antioxidant, anti-inflammatory, anticarcinogenic, antithrombotic, cytoprotective and vasoprotective activities, which are beneficial for oral compositions. Further, rutin augments antiplaque and antioxidant activity in oral compositions.

Non-limiting examples of antibacterial, antioxidant, and/or anti-inflammatory natural extracts include those isolated from green or oolong tea, cinnamon, gold thread, cranberry and other Ericaceae family plants, honeysuckle, grape seed, myrobalan, rosemary, east Indian walnut, neem, niruri, and pine bark.

Green tea and oolong tea are isolated from *Camellia sinensis.* Any variety, form, or subspecies of *Camellia sinensis* may be used and these may be selected from any subspecific taxon thereof, suitable examples of which are: *C. sinensis var. assamica,* which includes, e.g., the former *C. assamica* and var. *kucha; C. sinensis var. cambodiensis,* which includes, e.g., the former subspecies *lasiocalyx* and var. *Shan*; *C. sinensis var. dehungensis; C. sinensis var. pubilimba;* and *C. sinensis var. sinensis,* which includes, e.g., the former vars. *bohea, macrophylla, parvifolia,* and *waldenae.* The active components of *Camellia sinensis* extracts are believed to be the polyphenol catechines including catechin, epocatechin, epigallocatechin, epicatchin gallate, gallocatechin and epigallocatechin. Extracts of unoxidized *camellia* (e.g., green tea) used in oral compositions are described in U.S. Patent Publication No. 2006/0141073 to Worrell and extracts of oxidized camellia (e.g., oolong tea) are in U.S. Patent Publication No. 2006/0141039 to Boyd, et al.*,* both assigned to Colgate-Palmolive. An example of a suitable *Camellia* extract is "Green Tea Extract CG," specification no. MS-0726-01, available from Sabinsa Corp.

Gold thread extracts may be obtained from one or more of the following plant families *Annonaceae, Berberidaceae, Menispermaceae, Papaveraceae, Ranunculaceae, Rutaceae, Zingiberaceae, Nadina, Mahonia,* and *Thalictrum spp.* For example, a gold thread extract having desirable advantages in an oral care composition is Coptis teeta (coptis). The active compound of gold thread extracts is believed to be berberine (an anti-inflammatory, anti-microbial compound). *Goldenseal* (Orange-root), *Hydrastis canadensis,* is of the family *Ranunculaceae,* and one of its active components is believed to be berberine, as well as hydrastine alkaloids. Other extracts having berberine as an active compound include *Mahonia aquifolium* (Oregon grape), *Phellodendron amurense* (phellodendron), *Berberis vulgaris* (barberry), and *Xanthorhiza simplicissima* (yellow root).

Honeysuckle (*Lonicera ceprifolium*) extracts may be obtained from the flower of the honeysuckle plant. The active polyphenol materials in the honeysuckle extract are believed to be the chlorogenic acid and/or lutenolin flavonoids. The *Ericaceae* family broadly refers to over 100 genera and the over 4,000 associated species, such as those disclosed in U.S. Pat. No. 5,980,869 to Sanker, et al. In certain embodiments, extracts from plants in the *Vaccinium* genus are useful as antibacterial natural extracts, such as cranberry (*Vaccinium macrocarpon*).

*Cinnamomum zeylanicum Nees* or *C. verum,* are believed to contain multiple active compounds including cinnamaldehyde, eugenol, ethyl cinnamate, beta-caryophyllene, linalool, and methyl chavicol. Extracts of cinnamon exhibit antioxidant and antibacterial activity. Grape seed or grape skin extracts are isolated from *Vitis Vinifera* plants and include various polyphenols, including resveratrol and antioxidant proanthocyanidins. *Myrobalan* is preferably extracted from *Terminalia Bellerica* fruit. Pine bark extract is preferably extracted from the cortex (bark) of *Pinus Pinaster* (Maritime pine), which includes pycnogenol and exhibits antibacterial, anti-inflammatory, antioxidant and anti-aging activities. The extract of the cortex of the neem or margosa plant (*Melia Azadirachta*) is a known antibacterial component. *Niruri* or *Phyllanthus Niruri* extract also is a known antibacterial extract. *Salvadora persica* (miswak) extract provides efficacious antibacterial effects in oral care compositions. In certain aspects, an additional natural extract may be isolated from *Paullinia cupana* (guarana), whose extract includes caffeine, catechins, theobromine, theophylline and other alkaloids.

*Piper betle* (betel) extract, especially extract derived from betel leaves, is believed to include active compounds such as chavibetol, chavicol, estragole, eugenol, methyl eugenol, and hydroxy catechol. *Syzygium aromaticum* (clove) extracts have antiseptic and anesthetic properties and include, for example, the compounds eugenol, beta-caryophylline, vanillin, crategolic acid, methyl salicylate, tannins, flavanoids (including eugenin, kaempferol, rhamnetin, and eugentitin), triterpenoids (such as oleanolic acid, stigmasterol and campesterol), and various sesquiterpenes. *Commiphora myrrha* (myrrh) is likewise useful in oral compositions to provide antimicrobial and anti-inflammatory benefits. Another suitable genera of plants is *Juglans,* including *Juglans regia* (Persian walnut or common walnut tree) whose extract has anti-inflammatory and antioxidant properties. Similarly, the leaf of East Indian walnut (*Albizia Lebbek*) is suitable for use as an extract.

In certain embodiments, the additional natural extract of the compositions described herein comprises at least one free-B-ring flavonoid. Flavonoids are a group of compounds including such classes of compounds as flavones, flavans, flavonols, dihydroflanonols, flavonones, and derivatives thereof. Free-B-ring flavonoids active ingredients for use in oral compositions are described in U.S. Patent Publication No. 2006/0140881 to Xu et al. and assigned to Colgate-Palmolive.

In various embodiments, the additional natural extract may comprise a free-B-ring flavonoid, which refers to a flavonoid compound that generally contains a 2,3-double bond and/or a 4-oxo group and lack any substituent groups on the aromatic B-ring. Such active ingredients for oral compositions are described in U.S. Patent Publication No. 2006/0140881 to Xu et al. and assigned to Colgate-Palmolive. Free-B-ring flavonoids can be isolated from plants of the family *Lamiaceae,* especially those of the subfamily *Scutellarioideae.* For example, the species *Scutellaria baicalensis* contains significant amounts of free-B-ring flavonoids, including baicalein, baicalin, wogonin, and baicalenoside. Free-B-ring flavonoids have antioxidant and anti-inflammatory properties and inhibit general activity of the cyclooxygenase enzyme COX-2. In certain aspects, the additional natural extract may optionally comprise either baicalin (also known by the Chinese name "Huangqingan"), 5,6-Dihydroxyflavone-7-O-glucoside, and baicalein (also known by the Chinese name "Huangqinsu"), 5,6,7-Trihydroxyflavone. In various embodiments, the additional natural extract of the oral compositions of the present disclosure may comprise baicalin, baicalein, or mixtures thereof.

Plants from the *Magnoliaceae* family, such as *Magnolia Officinalis* (magnolia) contain active compounds including: magnolol, honokiol, tetrahydromagnolol, and tetrahydrohonokiol, which have demonstrated bactericidal properties against various oral bacteria. In various aspects, either magnolol and/or honokiol are useful antibacterial botanical active ingredients. The use of active compounds from magnolia extract is described in U.S. Patent Publication Nos. 2006/0134024 to Trivedi et al., and 2006/0127329 to Xu et al., both assigned to Colgate-Palmolive.

Other suitable natural extracts that have known antimicrobial, antioxidant, and/or anti-inflammatory agents are those listed in the International Cosmetic Ingredient Dictionary and Handbook, Tenth Ed., 2004.

The *Zingiber officinale* extract can be prepared according to known methods by water or alcohol extraction of water or alcohol soluble components, or from freeze drying the root or rhizome of *Zingiber officinale.* Preferred extracts can be derived from the rhizome of the *Zingiber officinale* plant. Extraction of a solid or liquid material from a plant typically involves contacting the material with an appropriate solvent to remove the substance(s) desired to be extracted from the material. Where the material is solid, it is preferably dried and crushed or ground prior to contacting it with the solvent. Such an extraction may be carried out by conventional means known to one of skill in the art, for example, by using an extraction apparatus, such as a Soxhlet apparatus, which retains the solid material in a holder and allows the solvent to flow through the material; by blending the solvent and material together and then separating the liquid and solid phases or two immiscible liquid phases, such as by filtration or by settling and decanting. In various embodiments, the botanical active ingredients used in oral care compositions are of reproducible, stable quality and have microbiological safety.

One method of preparing an extract of *Zingiber officinale* including extracting the plant material with an extraction solvent such as methanol, ethanol, isopropanol, butanol, xylene, benzene, or toluene, and concentrating and crystallizing a crude product from the extraction solvent. While this product could be used as the extract, additional procedures may be useful in purifying certain extracted components. For example, the crude product can be dissolved in a diol and optionally one of the solvents described above, the dissolved crude product then can be distributed between the solvent phase and the diol phase. If one of the solvents described above were not added with the diol, then one or more of the solvents are added before distributing between the two phases, and if one of the solvents were added, more is added before the distribution process. The solvent phase is concentrated and from the concentrate that extract is recrystallized.

Another method of preparing an extract of *Zingiber officinale* is hot water extraction. The temperature conditions and time conditions for the hot water extraction are not particularly limited, and they may be general conditions for hot water extraction (e.g., general conditions for preparation of decoction; 30 min to 60 min extraction at the boiling temperature). The temperature is preferably 80°C-100°C, more preferably 90°C-95°C, and the time is preferably not less than 1 hr, more preferably not less than 2 hr, particularly preferably not less than 3 hr. The hot water extraction under such temperature conditions and time conditions are preferable in that a highly effective composition can be obtained. The amount of water used for the hot water extraction is not particularly limited, but it is generally 5 parts by weight-20 parts by weight of water, preferably 10 parts by weight of water, per 1 part by weight of the *Zingiber officinale.*

By concentrating the obtained extract (extract solution), unnecessary volatile components can be removed and a preparation less burdensome on the digestive organs and the like by oral administration of a large amount can be obtained. The extract is preferably concentrated under the atmospheric pressure or under reduced pressure at 50°C-90°C, more preferably under reduced pressure at 50°C-60°C, to a solid content concentration to 20 wt %-40 wt %, preferably 25 wt %-35 wt %.

Furthermore, by adding an excipient to the obtained concentrate and drying, a stable powder preparation can be obtained. The excipient is not particularly limited as long as it is acceptable as a food or pharmaceutical agent, such as starch (e.g., cornstarch, potatostarch, wheat starch, rice starch), glucose, fructose, sorbitol, mannitol, carboxymethyl cellulose, carboxymethyl cellulose calcium, lactose, sucrose, hydroxypropyl cellulose, magnesium carbonate, magnesium oxide, calcium phosphate and the like. The amount of addition of the excipient is generally 1 part by weight-20 parts by weight, preferably 2 parts by weight-10 parts by weight, per 1 part by weight of the concentrate. The drying is preferably conducted at a temperature of 60°C-70°C.

Another method of preparing an extract of *Zingiber officinale* is by an ethanol extraction procedure. In this method, dried, preserved material from the *Zingiber officinale* plant (preferably the root or rhizome) is blended in ethanol at a weight to volume ratio of 1:3. The mixture then can be filtered, the residue again mixed in fresh ethanol, (this process can be repeated 3-5 times, if desired), and the filtrates from each respective filtration, collected. The collected filtrates then can be dried to remove the solvent, for example, rotary evaporated at or 45 °C, and then freeze dried to completely dry the extract. The dried extract then can be used as the extract, or once again reconstituted in ethanol.

The extract of *Zingiber officinale* also may be prepared by a hot soxhalation method in a similar manner. For example, coarse powdered material of the rhizomes of *Zingiber officinale* can be subjected to hot soxhalation using solvents such as petroleum ether, n-hexane, dichloromethane, chloroform, ethyl alcohol, ethyl acetate, acetone and methanol, at optimum temperature and recycled until extraction was completed. The plant extracts then can be filtered and concentrated to dryness on rotatory evaporator or on steam bath at optimum temperature.

Extracts of *Zingiber officinale* also may be prepared from the root or rhizome of the plant by steam distilling dried rhizomes, and concentrating the resulting distillate by evaporation to obtain a crude extract (which then can be further processed, as described above). In addition, extracts of *Zingiber officinale* can be obtained by extracting powder of dried rhizomes with supercritical CO₂, recovering the resulting extraction solution of the supercricital CO₂, and evaporating CO₂ from the extraction solution to obtain a crude extract.

The components of the extract of *Zingiber officinale* can be determined by subjecting the extracts to HPTLC (High Performance Thin Layer Chromatography) and HPLC (High performance Liquid chromatography) and Gas Chromatography (GC) in various mobile phases on precoated TLC plates (Merck), ODS column and 10% Carbowax 20M (2 meter) GC column (Temp. 70-220°C) respectively for qualitative and quantitative estimation of marker compounds and active principles. The extract preferably contains one or more of the following: bisaboline, cineol, phelladrene, citral, borneal, citronellal, geramial, linalool, limonene, zingiberol, zingiberine, camphene, gingerol, shogaol, zingerone, zingibain, vitamin B6, vitamin C, calcium, magnesium, phosphorus, potassium, linoleic acid, pectic polysaccharides (rhammose, arabinose, xylose, mannose, galactose, glucose and the like), gallic acid, tannic acid, gentisic acid, protocatechuic acid, Vanillic acid, caffeic acid, syringic acid, cinnamic acid, and mixtures thereof, and mixtures thereof.

Other methods of preparing an extract of *Zingiber officinale* will be readily apparent to those skilled in the art, upon review of the description herein.

Treatment levels of the components in various oral compositions are chosen to deliver an effective amount of the extract of *Zingiber officinale* to the oral surfaces of the subject animal in which the oral compositions are applied. For example, in toothpaste and tooth gels, suitable concentrations of the combination of extracts described herein include 0.01% by weight to 5% by weight, for example 0.05-5% by weight, and particularly 0.1-0.3% by weight.

For tooth powders, the treatment levels are approximately the same as for toothpastes and gels, while for rinses and washes, the treatment levels tend to be less. For example, mouth rinses and mouth washes contain 0.01% to 2% by weight of the combination of extracts, for example from 0.01% to 0.6%, 0.01% to 0.2%, and 0.01 to 0.05%. In addition, chewing gum, paint-on compositions, edible strips, and the like tend to be formulated with a wide range of concentration of extracts. In various embodiments, the level of extracts is similar to those in mouth rinses.

In one aspect, addition of the combination of extracts at the treatment levels discussed above with respect to various oral compositions has the effect of adding the major component(s) of extract of *Zingiber officinale,* such as bisaboline, cineol, phelladrene, citral, borneal, citronellal, geramial, linalool, limonene, zingiberol, zingiberine, camphene, gingerol, shogaol, dehydrogingerdione, zingerone, zingibain, vitamin B6, vitamin C, calcium, magnesium, phosphorus, potassium, linoleic acid, pectic polysaccharides (rhammose, arabinose, xylose, mannose, galactose, glucose and the like), gallic acid, tannic acid, gentisic acid, protocatechuic acid, Vanillic acid, caffeic acid, syringic acid, cinnamic acid, and mixtures and derivatives thereof, at treatment levels that are reduced from those given above by the percent by weight composition made up of the individual components. Thus, in one embodiment, the invention provides dentifrices comprising gingerol, shogaol, dehydrogingerdione, pectic polysaccharides, or mixtures thereof, in oral compositions at treatment levels of 0.01% by weight to 5% by weight.

In various embodiments, the compositions are formulated containing at least one humectant, at least one abrasive material, a carrier, and an effective amount of a combination of extracts. In one embodiment, the compositions contain 0.01% to 5% by weight of the combination of extracts, preferably 0.1 % to 2% by weight of the combination of extracts. In various preferred embodiments, the compositions contain 1% to 70% by weight of at least one humectant, and 1% to 70% by weight of at least one abrasive material, in addition to 0.1% to 2% by weight of the combination of extracts.

In various embodiments, the compositions do not include additional antibacterial agents, although their use is optional. In the event additional antibacterial agents are used, the compositions may further comprise an antibacterial agent selected from the group consisting of cetyl pyridinium chloride, polyphenols, phenolic compounds, stannous ions, zinc ions, and the like.

Te compositions described herein may be formulated with optional other ingredients, including without limitation anticaries agent, anticalculus or tartar control agents, anionic carboxylate polymers, viscosity modifiers, surfactants, flavorants, pigments, signals (flavor, color, light, heat, smell and other signals that signal the efficacious or advantageous use of the composition), agents to treat dry mouth, and the like.

In various embodiments, the compositions comprise an orally acceptable source of fluoride ions, which serves as an anticaries agent. One or more such sources can be present. Suitable sources of fluoride ions include fluoride, monofluorophosphate and fluorosilicate salts as well as amine fluorides, including olaflur (N'-octadecyltrimethylendiamine-N,N,N'- tris(2-ethanol)-dihydrofluoride).

As anticaries agent, one or more fluoride-releasing salts are optionally present in an amount providing a total of 100 to 20,000 ppm, 200 to 5,000 ppm, or 500 to 2,500 ppm, fluoride ions. Where sodium fluoride is the sole fluoride-releasing salt present, illustratively an amount of 0.0 1 % to 5%, 0.05% to 1% or 0.1% to 0.5%, sodium fluoride by weight can be present in the composition. Other anticaries agents can be used, such as arginine and arginine derivatives (e.g., ethyl lauroyl arginine (ELAH)).

Phenolic compounds useful herein illustratively include, subject to determination of oral acceptability, those identified as having anti-inflammatory activity by Dewhirst (1980), Prostaglandins 20(2), 209-222, but are not limited thereto. Examples of antibacterial phenolic compounds include 4-allylcatechol, *p*-hydroxybenzoic acid esters including benzylparaben, butylparaben, ethylparaben, methylparaben and propylparaben, 2-benzylphenol, butylated hydroxyanisole, butylated hydroxytoluene, capsaicin, carvacrol, creosol, eugenol, guaiacol, halogenated bisphenolics including hexachlorophene and bromochlorophene, 4-hexylresorcinol, 8-hydroxyquinoline and salts thereof, salicylic acid esters including menthyl salicylate, methyl salicylate and phenyl salicylate, phenol, pyrocatechol, salicylanilide, and thymol. These phenolic compounds typically are present in one or more of the natural extracts described above.

The at least one phenolic compound is optionally present in a total amount of 0.01% to 10% by weight. Illustratively the total concentration of the at least one phenolic compound in a toothpaste or gel dentifrice or mouth rinse of the present invention can be 0.01 % to 5%, for example 0.1% to 2%, 0.2% to 1% or 0.25% to 0.5%.

Other suitable antibacterial agents include, without limitation, copper (II) compounds such as copper (II) chloride, fluoride, sulfate and hydroxide, zinc ion sources such as zinc acetate, zinc citrate, zinc gluconate, zinc glycinate, zinc oxide, zinc sulfate and sodium zinc citrate, phthalic acid and salts thereof such as magnesium monopotassium phthalate, hexetidine, octenidine, sanguinarine, benzalkonium chloride, domiphen bromide, alkylpyridinium chlorides such as cetylpyridinium chloride (CPC) (including combinations of CPC with zinc and/or enzymes), tetradecylpyridinium chloride and N-tetradecyl-4-ethylpyridinium chloride, iodine, sulfonamides, bisbiguanides such as alexidine, chlorhexidine and chlorhexidine digluconate, piperidino derivatives such as delmopinol and octapinol, magnolia extract, grapeseed extract, menthol, geraniol, citral, eucalyptol, antibiotics such as augmentin, amoxicillin, tetracycline, doxycycline, minocycline, metronidazole, neomycin, kanamycin and clindamycin, and the like. A further illustrative list of useful antibacterial agents is provided in U.S. Patent No. 5,776,435 to Gaffar et al. If present, these additional antimicrobial agents are present in an antimicrobial effective total amount, typically 0.05% to 10%, for example 0.1 % to 3% by weight, of the composition.

In another embodiment the composition comprises an orally acceptable anticalculus agent. One or more such agents can be present. Suitable anticalculus agents include without limitation phosphates and polyphosphates (for example pyrophosphates), polyaminopropanesulfonic acid (AMPS), zinc citrate trihydrate, polypeptides such as polyaspartic and polyglutamic acids, polyolefin sulfonates, polyolefin phosphates, diphosphonates such as azacycloalkane-2,2-diphosphonates (*e.g.*, azacycloheptane-2,2-diphosphonic acid), N-methyl azacyclopentane-2,3-diphosphonic acid, ethane-1-hydroxy-1,1-diphosphonic acid (EHDP) and ethane-1-amino-1,1-diphosphonate, phosphonoalkane carboxylic acids and salts of any of these agents, for example their alkali metal and ammonium salts. Useful inorganic phosphate and polyphosphate salts illustratively include monobasic, dibasic and tribasic sodium phosphates, sodium tripolyphosphate, tetrapolyphosphate, mono-, di-, tri- and tetrasodium pyrophosphates, disodium dihydrogen pyrophosphate, sodium trimetaphosphate, sodium hexametaphosphate and the like, wherein sodium can optionally be replaced by potassium or ammonium. Other useful anticalculus agents include anionic polycarboxylate polymers. The anionic polycarboxylate polymers contain carboxyl groups on a carbon backbone and include polymers or copolymers of acrylic acid, methacrylic, and maleic anhydride. Non-limiting examples include polyvinyl methyl ether/maleic anhydride (PVME/MA) copolymers, such as those available under the Gantrez™ brand from ISP, Wayne, NJ. Still other useful anticalculus agents include sequestering agents including hydroxycarboxylic acids such as citric, fumaric, malic, glutaric and oxalic acids and salts thereof, and aminopolycarboxylic acids such as ethylenediaminetetraacetic acid (EDTA). One or more anticalculus agents are optionally present in the composition in an anticalculus effective total amount, typically 0.01% to 50%, for example 0.05% to 25% or 0.1% to 15% by weight.

In various embodiments, the anticalculus system comprises a mixture of sodium tripolyphosphate (STPP) and a tetrasodium pyrophosphate (TSPP). In various embodiments, the ratio of TSPP to STPP ranges 1:2 to 1:4. In a preferred embodiment, the first anticalculus active ingredient, TSPP is present at 1 to 2.5% and the second anticalculus active ingredient, STPP is present at 1 to 10%.

In one embodiment, the anionic polycarboxylate polymer is present 0.1% to 5%. In another embodiment, the anionic polycarboxylate polymer is present 0.5% to 1.5%, most preferably at 1% of the oral care composition. In one embodiment according to the present invention, the anticalculus system comprises a copolymer of maleic anhydride and methyl vinyl ether, such as for example, the Gantrez S-97 product discussed above.

In various embodiments, the ratio of TSPP to STPP to the synthetic anionic polycarboxylate ranges 5:10:1 to 5:20:10 (or 1:4:2). In one embodiment, the anticalculus system of the oral care composition comprises TSPP, STPP, and a polycarboxylate such as a copolymer of maleic anhydride and methyl vinyl ether at a ratio of 1:7:1. In a non-limiting embodiment, the anticalculus system consists essentially of TSPP present at 0.5% to 2.5%, STPP present at 1% to 10%, and a copolymer of maleic anhydride and methyl vinyl ether present at 0.5% to 1.5%

In another embodiment the composition comprises an orally acceptable stannous ion source useful, for example, in helping reduce gingivitis, plaque, calculus, caries or sensitivity. One or more such sources can be present. Suitable stannous ion sources include without limitation stannous fluoride, other stannous halides such as stannous chloride dihydrate, stannous pyrophosphate, organic stannous carboxylate salts such as stannous formate, acetate, gluconate, lactate, tartrate, oxalate, malonate and citrate, stannous ethylene glyoxide and the like. One or more stannous ion sources are optionally and illustratively present in a total amount of 0.01 % to 10%, for example 0.1% to 7% or 1% to 5% by weight of the composition.

In another embodiment the composition comprises an orally acceptable zinc ion source useful, for example, as an antimicrobial, anticalculus or breath-freshening agent. One or more such sources can be present. Suitable zinc ion sources include without limitation zinc acetate, zinc citrate, zinc gluconate, zinc glycinate, zinc oxide, zinc sulfate, sodium zinc citrate and the like. One or more zinc ion sources are optionally and illustratively present in a total amount of 0.05% to 3%, for example 0.1% to 1%, by weight of the composition.

In another embodiment the composition comprises an orally acceptable breath-freshening agent. One or more such agents can be present in a breath-freshening effective total amount. Suitable breath-freshening agents include without limitation zinc salts such as zinc gluconate, zinc citrate and zinc chlorite, α-ionone and the like.

In another embodiment the composition comprises an orally acceptable antiplaque, including plaque disrupting, agent. One or more such agents can be present in an antiplaque effective total amount. Suitable antiplaque agents include without limitation stannous, copper, magnesium and strontium salts, dimethicone copolyols such as cetyl dimethicone copolyol, papain, glucoamylase, glucose oxidase, urea, calcium lactate, calcium glycerophosphate, strontium polyacrylates and chelating agents such as citric and tartaric acids and alkali metal salts thereof.

In another embodiment the composition comprises an orally acceptable anti-inflammatory agent other than the rosemary components described above. One or more such agents can be present in an anti-inflammatory effective total amount. Suitable anti-inflammatory agents include without limitation steroidal agents such as flucinolone and hydrocortisone, and nonsteroidal agents (NSAIDs) such as ketorolac, flurbiprofen, ibuprofen, naproxen, indomethacin, diclofenac, etodolac, indomethacin, sulindac, tolmetin, ketoprofen, fenoprofen, piroxicam, nabumetone, aspirin, diflunisal, meclofenamate, mefenamic acid, oxyphenbutazone and phenylbutazone. One or more anti-inflammatory agents are optionally present in the composition in an anti-inflammatory effective amount.

Compositions of the inventions optionally contain other ingredients such as enzymes, vitamins and anti-adhesion agents. Enzymes such as proteases can be added for anti-stain and other effects. Non-limiting examples of vitamins include vitamin C, vitamin E, vitamin B5, and folic acid. In various embodiments, the vitamins have antioxidant properties. Anti-adhesion agents include ethyl lauroyl arginine (ELAH), solbrol, ficin, silicone polymers and derivatives, and quorum sensing inhibitors.

Among useful carriers for optional inclusion in a composition of the invention are diluents, abrasives, bicarbonate salts, pH modifying agents, surfactants, foam modulators, thickening agents, viscosity modifiers, humectants, sweeteners, flavorants and colorants. One carrier material, or more than one carrier material of the same or different classes, can optionally be present. Carriers should be selected for compatibility with each other and with other ingredients of the composition.

Water is a preferred diluent and in some compositions such as mouthwashes and whitening liquids is commonly accompanied by an alcohol, *e.g*., ethanol. The weight ratio of water to alcohol in a mouthwash composition is generally 1:1 to 20:1, for example 3:1 to 20:1 or 4:1 to 10:1. In a whitening liquid, the weight ratio of water to alcohol can be within or below the above ranges, for example 1:10 to 2:1.

In one embodiment a composition of the invention comprises at least one abrasive, useful for example as a polishing agent. Any orally acceptable abrasive can be used, but type, fineness (particle size) and amount of abrasive should be selected so that tooth enamel is not excessively abraded in normal use of the composition. Suitable abrasives include without limitation silica, for example in the form of silica gel, hydrated silica or precipitated silica, alumina, insoluble phosphates, calcium carbonate, resinous abrasives such as urea-formaldehyde condensation products and the like. Among insoluble phosphates useful as abrasives are orthophosphates, polymetaphosphates and pyrophosphates. Illustrative examples are dicalcium orthophosphate dihydrate, calcium pyrophosphate, β-calcium pyrophosphate, tricalcium phosphate, calcium polymetaphosphate and insoluble sodium polymetaphosphate. One or more abrasives are optionally present in an abrasive effective total amount, typically 5% to 70%, for example 10% to 50% or 15% to 30% by weight of the composition. Average particle size of an abrasive, if present, is generally 0.1 to 30 µm, for example 1 to 20 µm or 5 to 15 µm.

In a further embodiment a composition of the invention comprises at least one bicarbonate salt, useful for example to impart a "clean feel" to teeth and gums due to effervescence and release of carbon dioxide. Any orally acceptable bicarbonate can be used, including without limitation alkali metal bicarbonates such as sodium and potassium bicarbonates, ammonium bicarbonate and the like. One or more bicarbonate salts are optionally present in a total amount of 0.1% to 50%, for example 1% to 20% by weight of the composition.

In a still further embodiment a composition of the invention comprises at least one pH modifying agent. Such agents include acidifying agents to lower pH, basifying agents to raise pH and buffering agents to control pH within a desired range. For example, one or more compounds selected from acidifying, basifying and buffering agents can be included to provide a pH of 2 to 10, or in various illustrative embodiments 2 to 8, 3 to 9, 4 to 8, 5 to 7, 6 to 10, 7 to 9, *etc.* Any orally acceptable pH modifying agent can be used, including without limitation carboxylic, phosphoric and sulfonic acids, acid salts (*e.g*., monosodium citrate, disodium citrate, monosodium malate, *etc*.), alkali metal hydroxides such as sodium hydroxide, carbonates such as sodium carbonate, bicarbonates, sesquicarbonates, borates, silicates, phosphates (*e.g*., monosodium phosphate, trisodium phosphate, pyrophosphate salts, *etc*.), imidazole and the like. One or more pH modifying agents are optionally present in a total amount effective to maintain the composition in an orally acceptable pH range.

In a still further embodiment a composition of the invention comprises at least one surfactant, useful for example to compatibilize other components of the composition and thereby provide enhanced stability, to help in cleaning the dental surface through detergency, and to provide foam upon agitation, *e.g*., during brushing with a dentifrice composition of the invention. Any orally acceptable surfactant, most of which are anionic, nonionic or amphoteric, can be used. Suitable anionic surfactants include without limitation water-soluble salts of C₈₋₂₀ alkyl sulfates, sulfonated monoglycerides of C₈₋₂₀ fatty acids, sarcosinates, taurates and the like. Illustrative examples of these and other classes include sodium lauryl sulfate, sodium coconut monoglyceride sulfonate, sodium lauryl sarcosinate, sodium lauryl isoethionate, sodium laureth carboxylate and sodium dodecyl benzenesulfonate. Suitable nonionic surfactants include without limitation poloxamers, polyoxyethylene sorbitan esters, fatty alcohol ethoxylates, alkylphenol ethoxylates, tertiary amine oxides, tertiary phosphine oxides, dialkyl sulfoxides and the like. Suitable amphoteric surfactants include without limitation derivatives of C₈₋₂₀ aliphatic secondary and tertiary amines having an anionic group such as carboxylate, sulfate, sulfonate, phosphate or phosphonate. A suitable example is cocoamidopropyl betaine. One or more surfactants are optionally present in a total amount of 0.0 1 % to 10%, for example 0.05% to 5% or 0.1 % to 2% by weight of the composition.

In a still further embodiment a composition of the invention comprises at least one foam modulator, useful for example to increase amount, thickness or stability of foam generated by the composition upon agitation. Any orally acceptable foam modulator can be used, including without limitation polyethylene glycols (PEGs), also known as polyoxyethylenes. High molecular weight PEGs are suitable, including those having an average molecular weight of 200,000 to 7,000,000, for example 500,000 to 5,000,000 or 1,000,000 to 2,500,000. One or more PEGs are optionally present in a total amount of 0.1% to 10%, for example 0.2% to 5% or 0.25% to 2% by weight of the composition.

In a still further embodiment a composition of the invention comprises at least one thickening agent, useful for example to impart a desired consistency and/or mouth feel to the composition. Any orally acceptable thickening agent can be used, including without limitation carbomers, also known as carboxyvinyl polymers, carrageenans, also known as Irish moss and more particularly 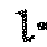 -carrageenan (iota-carrageenan), cellulosic polymers such as hydroxyethylcellulose, carboxymethylcellulose (CMC) and salts thereof, *e.g*., CMC sodium, natural gums such as karaya, xanthan, gum arabic and tragacanth, colloidal magnesium aluminum silicate, colloidal silica and the like. One or more thickening agents are optionally present in a total amount of 0.01% to 15%, for example 0.1% to 10% or 0.2% to 5% by weight of the composition.

In a still further embodiment a composition of the invention comprises at least one viscosity modifier, useful for example to inhibit settling or separation of ingredients or to promote redispersibility upon agitation of a liquid composition. Any orally acceptable viscosity modifier can be used, including without limitation mineral oil, petrolatum, clays and organomodified clays, silica and the like. One or more viscosity modifiers are optionally present in a total amount of 0.0 1 % to 10%, for example 0.1% to 5% by weight of the composition.

In a still further embodiment a composition of the invention comprises at least one humectant, useful for example to prevent hardening of a tooth paste upon exposure to air. Any orally acceptable humectant can be used, including without limitation polyhydric alcohols such as glycerin, sorbitol, xylitol or low molecular weight PEGs. Most humectants also function as sweeteners. One or more humectants are optionally present in a total amount of 1% to 70%, for example 1% to 50%, 2% to 25%, or 5% to 15% by weight of the composition.

In a still further embodiment a composition of the invention comprises at least one sweetener, useful for example to enhance taste of the composition. Any orally acceptable natural or artificial sweetener can be used, including without limitation dextrose, sucrose, maltose, dextrin, dried invert sugar, mannose, xylose, ribose, fructose, levulose, galactose, corn syrup (including high fructose corn syrup and corn syrup solids), partially hydrolyzed starch, hydrogenated starch hydrolysate, sorbitol, mannitol, xylitol, maltitol, isomalt, aspartame, neotame, saccharin and salts thereof, dipeptide-based intense sweeteners, cyclamates and the like. One or more sweeteners are optionally present in a total amount depending strongly on the particular sweetener(s) selected, but typically 0.005% to 5% by weight of the composition.

In a still further embodiment a composition of the invention comprises at least one flavorant, useful for example to enhance taste of the composition. Any orally acceptable natural or synthetic flavorant can be used, including without limitation vanillin, sage, marjoram, parsley oil, spearmint oil, cinnamon oil, oil of wintergreen (methylsalicylate), peppermint oil, clove oil, bay oil, anise oil, eucalyptus oil, citrus oils, fruit oils and essences including those derived from lemon, orange, lime, grapefruit, apricot, banana, grape, apple, strawberry, cherry, pineapple, *etc.,* bean- and nut-derived flavors such as coffee, cocoa, cola, peanut, almond, *etc.,* adsorbed and encapsulated flavorants and the like. Also encompassed within flavorants herein are ingredients that provide fragrance and/or other sensory effect in the mouth, including cooling or warming effects. Such ingredients illustratively include menthol, menthyl acetate, menthyl lactate, camphor, eucalyptus oil, eucalyptol, anethole, eugenol, cassia, oxanone, α-irisone, propenyl guaiethol, thymol, linalool, benzaldehyde, cinnamaldehyde, N-ethyl-*p*-menthan-3-carboxamine, N,2,3-trimethyl-2-isopropylbutanamide, 3-(1-menthoxy)-propane-1,2-diol, cinnamaldehyde glycerol acetal (CGA), menthone glycerol acetal (MGA) and the like. One or more flavorants are optionally present in a total amount of 0.0 1 % to 5%, for example 0.1% to 2.5% by weight of the composition.

In a still further embodiment a composition of the invention comprises at least one colorant. Colorants herein include pigments, dyes, lakes and agents imparting a particular luster or reflectivity such as pearling agents. A colorant can serve a number of functions, including for example to provide a white or light-colored coating on a dental surface, to act as an indicator of locations on a dental surface that have been effectively contacted by the composition, and/or to modify appearance, in particular color and/or opacity, of the composition to enhance attractiveness to the consumer. Any orally acceptable colorant can be used, including without limitation talc, mica, magnesium carbonate, calcium carbonate, magnesium silicate, magnesium aluminum silicate, silica, titanium dioxide, zinc oxide, red, yellow, brown and black iron oxides, ferric ammonium ferrocyanide, manganese violet, ultramarine, titaniated mica, bismuth oxychloride and the like. One or more colorants are optionally present in a total amount of 0.00 1 % to 20%, for example 0.01 % to 10% or 0.1 % to 5% by weight of the composition.

In another embodiment, mouthwash or mouth rinse compositions are provided that contain water, one or more flavorants such as discussed above, one or more organic hydric compounds, and an antibacterial effective amount of an antibacterial composition as discussed above. In various embodiments, the mouthwash or mouth rinse compositions contain from 0.001% to 5% by weight of an alcohol extract of the leaves of a plant containing ursolic acid and carnosic acid, such as *Rosmarinus. officinalis.* In preferred embodiments, the compositions contain 0.01% to 1% by weight of rosemary extract, for example 0.02% to 0.5% by weight The one or more organic hydric compounds are orally acceptable organic solvents such as, without limitation, ethanol and glycerol. Optionally, the mouthwash and mouth rinse compositions contain a surfactant to aid in dispersal of the flavorants and antibacterial compositions.

In various embodiments, the invention provides chewing gum compositions comprising a gum base and an effective amount of the combination of extracts discussed above. Chewing gum formulations typically contain, in addition, one or more plasticizing agents, at least one sweetening agent and at least one flavoring agent.

Gum base materials are well known in the art and include natural or synthetic gum bases or mixtures thereof. Representative natural gums or elastomers include chicle, natural rubber, jelutong, balata, guttapercha, lechi caspi, sorva, guttakay, crown gum, and perillo. Synthetic gums or elastomers include butadiene-styrene copolymers, polyisobutylene and isobutylene-isoprene copolymers. The gum base is incorporated in the chewing gum product at a concentration of 10 to 40% and preferably 20 to 35%.

In other embodiments, the oral compositions comprise an edible oral strip comprising one or more polymeric film forming agents and an effective amount of the combination of extracts discussed above. The one or more polymeric film forming agents are selected from the group consisting of orally acceptable polymers such as pullulan, cellulose derivatives, and other soluble polymers including those well-known in the art.

In various embodiments, the compositions are effective against a combination of oral bacteria, as shown for example, in artificial mouth antiplaque study. In various embodiments, significant reductions in plaque development are seen in comparison to a negative control containing none of the antibacterial composition.

In various embodiments, the compositions also show antioxidant properties, for example as demonstrated in an LPO-CC assay carried out with formulated dentifrices, and/or also show clinical effectiveness *in vivo.* For example, in preferred embodiments, compositions of the invention show anti-gingival efficacy in a modified gingival margin plaque index determination. The protocol, known as MGMPI, has been published. Compositions including rosemary extract at an effective amount show significant improvements over a negative control. In other embodiments, compositions of the invention are also effective against plaque as shown in short-term clinical studies.

In various embodiments, the invention is based in part on the discovery that when components such as found in extracts of *Zingiber officinale* are added to dentifrice compositions, the anti-inflammatory effect of the dentifrice composition is enhanced. Accordingly, the invention provides in various embodiments dentifrice compositions that contain a combination of extracts, including an extract of *Zingiber officinale,* and a natural extract other than *Zingiber officinale.*

The preferred embodiments now will be described in more detail with reference to the following non-limiting examples.

### EXAMPLES

### Example 1

A toothpaste formulation is prepared using the following ingredients:

**Table 1. Zingiber officinale dentifrice**

| **Ingredient** | **Grams (as supplied)** |
|---|---|
| Purified Water | Q. S. |
| Sodium Saccharin | 0.3 |
| Sodium Fluoride | 0.243 |
| 70% Sorbitol - Non Browning | 20.85 |
| 99.0% Glycerin | 20 |
| Sodium CMC | 1.1 |
| Iota Carrageenan | 0.4 |
| Titanium Dioxide | 0.5 |
| 13% Liquid Gantrez polymer | 15 |
| Sodium Hydroxide (NaOH) | 1.2 |
| Zeodent™ 115 silica | 20 |
| Zeodent™ 165 silica | 1.5 |
| Flavor K91-4778 | 1 |
| 30% Liquid sodium lauryl sulfate | 5.172 |
| Additional natural extract | 0.3 |
| *Garcinia mangostana L* extract | 0.1 - 0.3 |

The above toothpaste formulation will provide improved antibacterial and anti-inflammatory properties, when compared to conventional toothpaste formulation without the combination of natural extracts. For example, the additional natural extract will be magnolia, rosemary, *Camellia,* morin, *Myristica fragrans, Oolong tea, Juglans regia, Zanthoxylum alantum, Mimusops elengi, Hibiscus abelmoschus, Ayurvedic, Garcinia mangostana L., Carapa procera, Khaya senegalensis, Salvadora persica,Cucurbitaceae (Citrullus colocynthis), Acacia catechu, Acacia nilotica, Achyrathes aspera, Azadirachta indica, Aristolochia bracteolate, Cinnamomum camphora, Cinnamomum verum, Curcuma longa, Eucalyptus globulus, Ficus bengalensis, Juglans regia, Zizyphus joazeiro, Madhuca longifolia, Mimusops elengi, Ocimum sanctum,* Oolonga tea, Piper betel leaves, *Piper longum, Piper nigrum, Potentilla fulgens, Syzygium aromaticum, Spilanthes calva, Vaccinium macrocarpon, Zanthoxylum armatum,* and the composition will have improved antibacterial and anti-inflammatory efficacy, when compared to toothpaste formulations that do not contain a combination of natural extracts and *Zizyphus joazeiro.*

### Example 2

A mouth wash formulation is prepared using the following ingredients:

**Table 2 - Zingiber officinale Mouthwash**

| **Component** | **% wt.** |
|---|---|
| Sucralose | 0.02 or less |
| Sodium Fluoride | 0.05 |
| Sodium Benzoate | 0.11 |
| Glycerin | 7.5 |
| Sorbitol | 5.5 |
| Propylene Glycol | 5 |
| Pluronic™ F127 surfactant | 0.15 |
| Ethyl Alcohol | 6 |
| Additional natural extract | 0.15 |
| *Zingiber officinale* extract | 0.02 |
| Flavor | Varies |
| Color | varies |
| Water | Q.S. |

The above mouthwash formulation will provide improved antibacterial and anti-inflammatory properties, when compared to conventional mouthwash formulations without the combination of natural extracts.

The invention has been described above with reference to illustrative Examples, but it is to be understood that the invention is not limited to the disclosed embodiments. Alterations and modifications that would occur to one of skill in the art upon reading the specification are also within the scope of the invention, which is defined in the appended claims.

## Claims

1. An oral composition comprising:
a combination of extracts comprising an extract from *Zingiber officinale* and a natural extract other than the extract from *Zingiber officinale*; and
an orally acceptable carrier.

2. A composition according to claim 1, comprising 0.01% to 5% by weight of the combination of extracts.

3. A composition according to claim 2, comprising 0.1 % to 2% by weight of the combination of extracts.

4. A composition according to any preceding claim, wherein the natural extract other than the extract from *Zingiber officinale* is one or more natural extracts selected from the group consisting of extracts of oregano, magnolia, cranberry, rosemary, *Camellia,* morin, *Myristica fragrans, Zizyphus joazeiro, Punica granatum, Garcinia mangostana L.,* Jabara, *Azadirachta indica, Acacia, Oolong tea, Juglans regia, Zanthoxylum alantum, Mimusops elengi, Hibiscus abelmoschus, Ayurvedic, Carapa procera, Khaya senegalensis, Salvadora persica,Cucurbitaceae (Citrullus colocynthis), Acacia catechu, Acacia nilotica, Achyrathes aspera, Azadirachta indica, Aristolochia bracteolate, Cinnamomum camphora, Cinnamomum verum, Curcuma longa, Eucalyptus globulus, Ficus bengalensis, Juglans regia, Madhuca longifolia, Mimusops elengi, Ocimum sanctum,* Oolonga tea, Piper betel leaves, *Piper longum, Piper nigrum, Potentilla fulgens, Syzygium aromaticum, Spilanthes calva, Vaccinium macrocarpon, Zanthoxylum armatum,* and mixtures thereof.

5. A composition according to any preceding claim, further comprising an additional antibacterial agent selected from: phenolic compounds, stannous ions, zinc ions, and mixtures thereof.

6. A composition according to claim 5, wherein the zinc ions are provided by one or more zinc-containing compounds selected from the group consisting of zinc acetate, zinc citrate, zinc gluconate, zinc glycinate, zinc oxide, zinc sulfate, sodium zinc citrate, and mixtures thereof.

7. A composition according to any preceding claim, wherein the composition further comprises at least one additional component selected from the group consisting of humectants, abrasives, anticaries agents, anticalculus or tartar control agents, anionic carboxylate polymers, viscosity modifiers, surfactants, flavorants, pigments, and mixtures thereof.

8. A composition according to any preceding claim, wherein the composition is a dentifrice in a form selected from the group consisting of: powder; toothpaste or dental gel; a periodontal gel; a liquid suitable for painting a dental surface; a chewing gum; a dissolvable, partially dissolvable or non-dissolvable film or strip; a bead, a wafer; a wipe or towelette; an implant; a mouthrinse, a foam, and dental floss.

9. A method of treating a disease or condition of oral cavity soft tissue comprising administering to the oral cavity of a patient in need thereof, a composition according to any of claims 1 to 8.

10. The method according to claim 9, wherein the disease or condition is xerostomia.
